# EUROPEAN PATENT APPLICATION

(11) **EP 3 699 300 A1**
(43) Date of publication of application: **26.08.2020**
(21) Application number: 19382133.7
(22) Date of filing: 22.02.2019
(51) Int. Cl.: C12Q 1/6886

(54) **SEMINAL MIRNAS AS NON-INVASIVE BIOMARKERS FOR THE DIAGNOSIS AND/OR PROGNOSIS OF PROSTATE CANCER**

(71) Applicant: Institut d'Investigació Biomèdica de Bellvitge (IDIBELL), 08908 Hospitalet de Llobregat (ES)
(72) Inventor: LARRIBA BARTOLOME, Sara, 08908 Hospitalet de Llobregat (Barcelona) (ES); CASTELLS ESTEVE, Manel, 08908 Hospitalet de Llobregat (Barcelona) (ES); VIGUÉS JULIÀ, Francesc, 08908 Hospitalet de Llobregat (Barcelona) (ES)
(74) Representative: Herrero & Asociados, S.L.

(57) **Abstract**

The present invention relates to a group of miRNAs, namely, miR-142-3p, miR-142-5p, miR-223-3p, miR-342-3p, miR-374b-5p or combinations thereof, used as biomarkers in seminal fluid for the diagnosis and/or prognosis of prostate cancer. In addition, the invention refers to an *in vitro* method for the diagnosis of prostate cancer in a subject or for discriminating prostate cancer from BPH in said subject, as well as to an *in vitro* method for discriminating the grade of a tumor in a subject suffering from prostate cancer.

SEQUENCE LISTING

<110>  Institut d'Investigació Biomedica de Bellvitge (IDIBELL)

<120>  SEMINAL EXTRACELLULAR  MiRNAS AS NON-INVASIVE BIOMARKERS FOR THE
       DIAGNOSIS AND/OR PROGNOSIS OF PROSTATE CANCER

<130>  20181368

<160>  5

<170>  PatentIn version 3.5

<210>  1
<211>  23
<212>  RNA
<213>  Homo sapiens

<220>
<223>  sequence of miR-142-3p

<400>  1
uguaguguuu ccuacuuuau gga                                              23


<210>  2
<211>  21
<212>  RNA
<213>  Homo sapiens

<220>
<223>  sequence of miR-142-5p

<400>  2
cauaaaguag aaagcacuac u                                                21


<210>  3
<211>  22
<212>  RNA
<213>  Homo sapiens

<220>
<223>  sequence of miR-223-3p

<400>  3
ugucaguuug ucaaauaccc ca                                               22


<210>  4
<211>  23
<212>  RNA
<213>  Homo sapiens

<220>
<223>  sequence of miR-342-3p

<400>  4
ucucacacag aaaucgcacc cgu                                              23


<210>  5
<211>  22

<212>  RNA
<213>  Homo sapiens

<220>
<223>  sequence of miR-374b-5p

<400>  5
auauaauaca accugcuaag ug                                                22

## Description

### FIELD OF THE INVENTION

The present invention belongs to the field of prostate cancer diagnosis and prognosis, more specifically it relates to a group of miRNAs used as biomarkers in seminal fluid in the diagnosis and/or prognosis of prostate cancer. In addition, the invention refers to an *in vitro* method for the diagnosis of prostate cancer in a subject or for discriminating prostate cancer from BPH in said subject, as well as to an *in vitro* method for discriminating the grade of a tumor in a subject suffering from prostate cancer.

### BACKGROUND OF THE INVENTION

Prostate cancer (PCa) is the most prevalent type of malignant male cancer in Western countries and a major cause of cancer-related deaths. Detection is mainly carried out by the determination of levels of prostate-specific antigen (PSA) in blood and/or by physical examination of the prostate gland (digital rectal examination -DRE-). Suspicious results are evaluated in prostate tissue samples (transrectal or transperineal biopsy), essential to confirm the diagnosis and in which the severity or degree of affectation will be determined by means of the modified Gleason Score (GS) [Epstein JI et al: Am J Surg Pathol 2016, 40:244-252].

PCa is a slow-growing tumor and fortunately, only a minority of these patients has invasive PCa. However, it may not cause signs or symptoms in its early stages, so it is critical to specifically identify those patients with clinically significant PCa and at an early stage. Since its application in clinical practice, the screening of PCa based on the determination of PSA has allowed for better detection of the disease in the early stages, and therefore it has contributed to the reduction of mortality due to malignant prostate disease. However, the deficiencies of serum PSA as a biomarker are well documented [Roobol MJ et al. Nat Rev Urol 2013, 10:38-48]. Although specific for prostatic tissue, PSA has low cancer specificity [Oesterling JE et al J Urol 1991, 145:907-923]. Thus, PSA screening has resulted in an over-diagnosis of PCa, and in many unnecessary biopsies of benign disease; only about 30-40% of the biopsied men are diagnosed with PCa and specifically, in patients with PSA levels of 4 to 10 ng/ml, the detection rate of PCa was merely 20% or less thus defining the region as a "grey zone". Conversely, not all prostate cancers give rise to an elevated serum PSA concentration [Oesterling JE et al J Urol 1991, 145:907-923]. Additionally, serum PSA levels do not correlate with tumor aggressiveness, survival, or response to pharmacological treatments leading to over-treatment of indolent tumors. Given this context, there is a need of more specific non-invasive diagnostic biomarkers, either alone or in addition to PSA marker, that allow the identication PCa patients.

MicroRNAs (miRNAs) are conserved small non-coding RNAs (19-22 nucleotides) that act as negative post-transcriptional modulators of gene expression, affecting mRNA stability and translation by interacting with complementary sites on the 3'UTR of the target mRNAs. Studies have shown that the miRNAs have critical roles in a variety of biological processes such as: cell proliferation, differentiation, apoptosis and carcinogenesis [Bartel DP et al Cell 2004, 116:281-297; He L. et al Nat Rev Genet 2004, 5:522-531]. MiRNAs are becoming more and more relevant as biomarkers for the diagnosis or prognosis of different pathological conditions or diseases.

MiRNAs have actually been disclosed as biomarker of prostate disease. For instance, EP2634266 teaches a method for the diagnosis and prognosis of prostate disease in serum or plasma based on different miRNAs.

Semen or seminal fluid is another source of miRNA, which would allow for a non-invasive diagnosis. Furthermore, approximately 40% of semen is derived from prostatic tissue, so that its contents are most likely to contain prostate disease-specific derived molecules. Therefore, human seminal fluid is emerging as a very likely source of PCa-specific biomarkers [Roberts MJ et al. Korean J Urol 2011, 52:79-89; Selth LA. et al. Endocr Relat Cancer. 2014, 21:L17-21; Drabovich AP. et al Nat Rev Urol 2014, 11:278-288]. Interestingly, seminal plasma (SP) contains an extraordinary concentration of extracellular miRNAs, some of them specific to the reproductive glands where it originates, such as the prostate. Such extracellular miRNAs in biofluids, and specifically in semen, are very stable and resistant to endogenous ribonuclease activity due to their presentation, either contained in extracellular vesicles (EVs) such as exosomes [Valadi H. et al. Nat Cell Biol 2007, 9:654-659] or free/soluble in protein complexes bound with RNA-binding proteins [Li H. et al. PLoS One 2012, 7:e34566]. It has been calculated that, in mammals, each ejaculate contains trillions of exosomes, characterised by a high content of cholesterol and sphingomyelin, and a very complex protein composition.

Interestingly, some molecular studies have shown a fingerprint of altered miRNA expression in FFPE prostate tumor tissue compared with benign disease controls [Kristensen H. et al. Oncotarget 2016, 7:30760-30771]. Actually, some miRNAs as potential biomarkers of PCa in non-sperm cellular fraction of seminal fluid have already been proposed in the art [Selth LA. et al. Endocr Relat Cancer. 2014, 21:L17-21].

Now, the authors of the present invention have identified several miRNAs, preferably extracellular miRNAs, as biomarkers in seminal fluid with predictive value in the diagnosis and or prognosis of PCa.

### BRIEF DESCRIPTION OF THE FIGURES

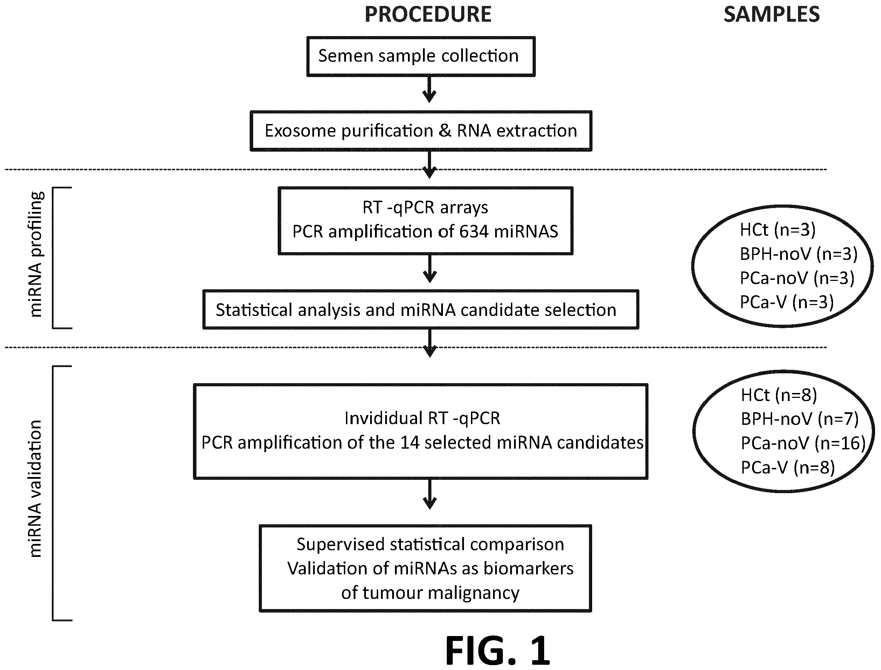

**Figure 1****:** Flow chart representing the two stages design study. The number of miRNAs and individuals analysed in each work-procedure stage are depicted.

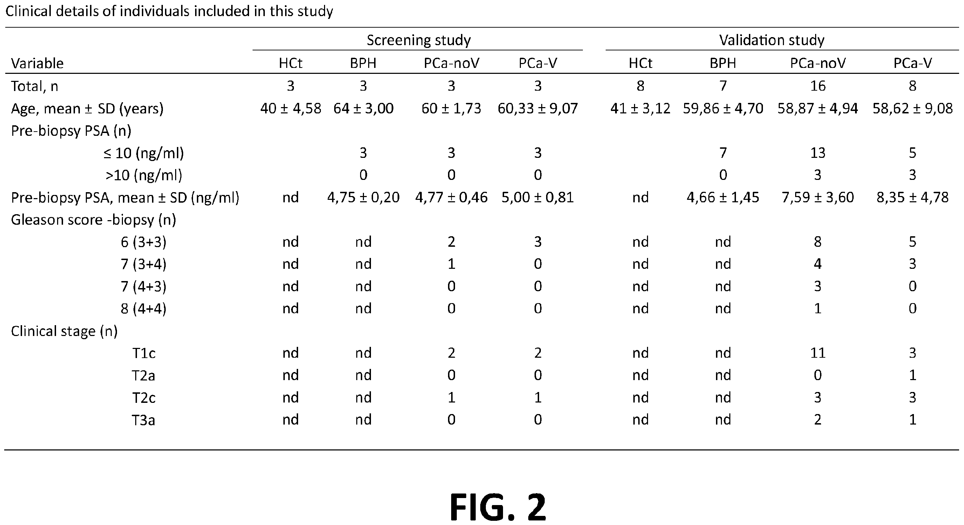

**Figure 2****:** Description of clinical details of the individuals of the study. The following nomenclature is used: HCt: healthy control group; BPH: benign prostate hyplerplasia group; PCa-noV: prostate cancer from non-vasectomized individuals; PCa-V: prostate cancer from vasectomized individuals.

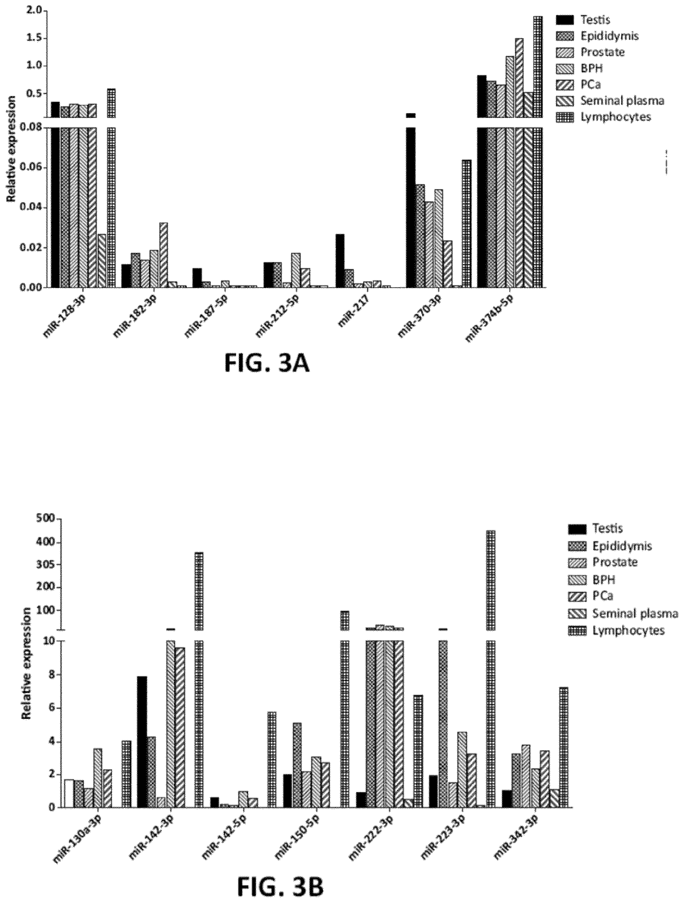

**Figure 3****:** Tissue expression profiling of the 14 candidate miRNAs (A and B). miRNA expression was determined by RT-qPCR in several reproductive organs such as testis, epididymis and prostate, as well as in exosomes from SP and lymphocytes. Controls of pathological prostate (BPH and PCa prostate) were also included. Expression levels relative to miR-30e-3p and miR-126-3p are shown.

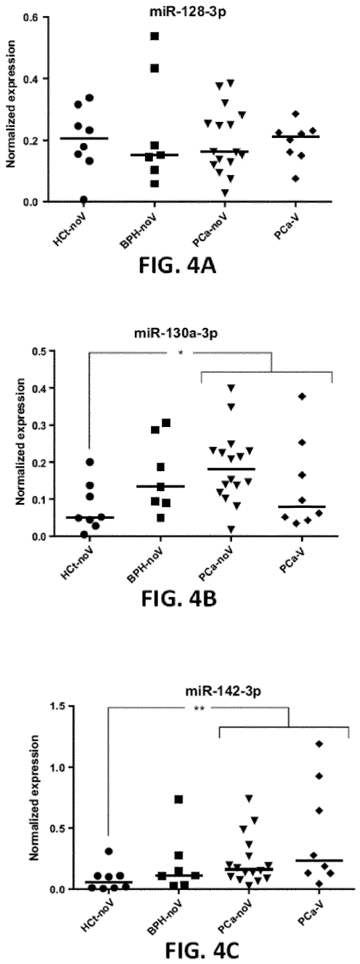

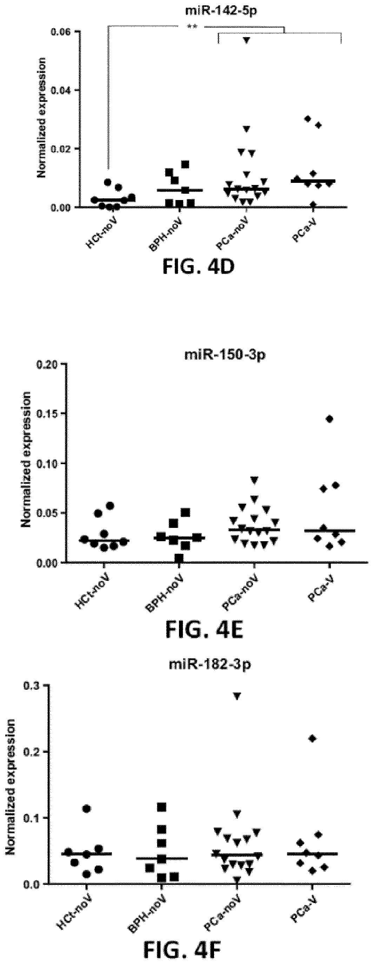

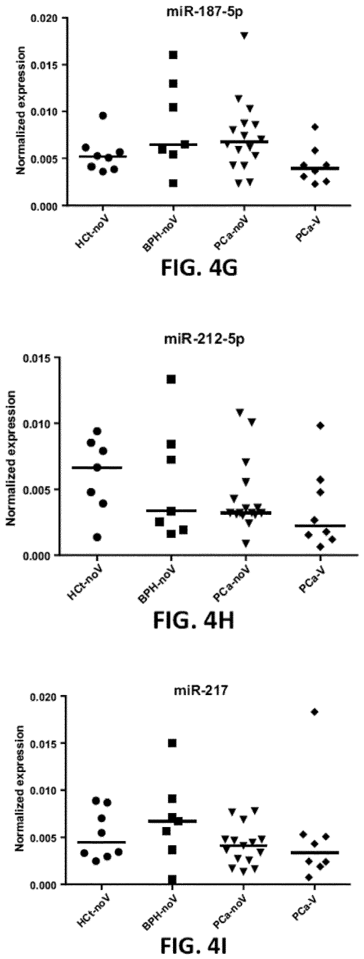

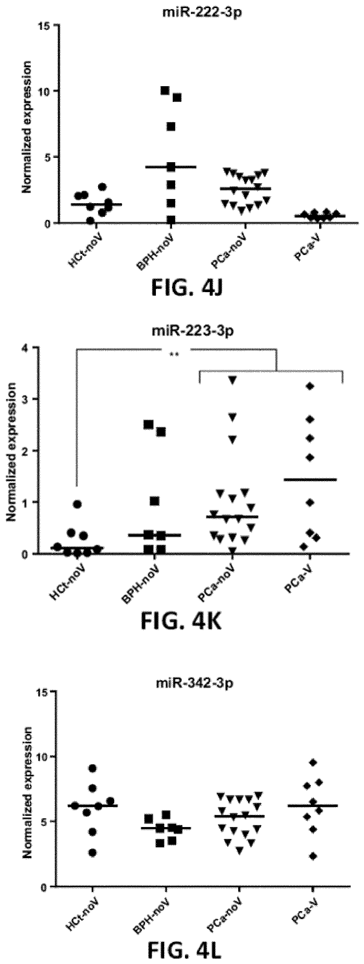

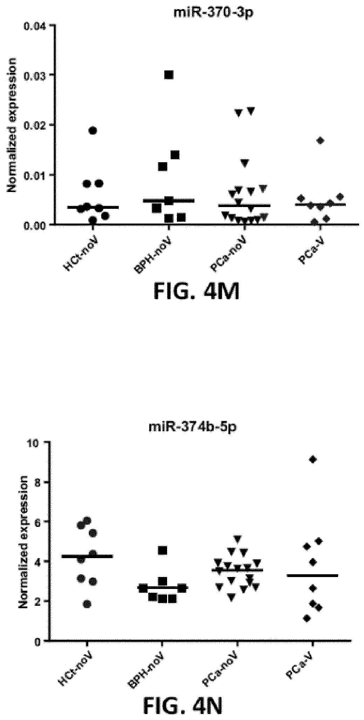

**Figure 4****:** Exosome miRNA levels in seminal plasma are altered in benign prostate hyperplasia and malignant prostate tumor. Expression profiling, at the validating stage, of the 14 miRNAs candidates (A to N) in exosomes from semen of healthy controls (HCt), benign prostate hyperplasia-non vasectomised (BPH-noV), prostate cancer-non vasectomised (PCa-noV) and prostate cancer from men successfully vasectomised (PCa-V). The horizontal bar displays the median cellular expression level. Significant differences between groups are indicated: *p<0.05; **p<0.01 (Mann Whitney U test).

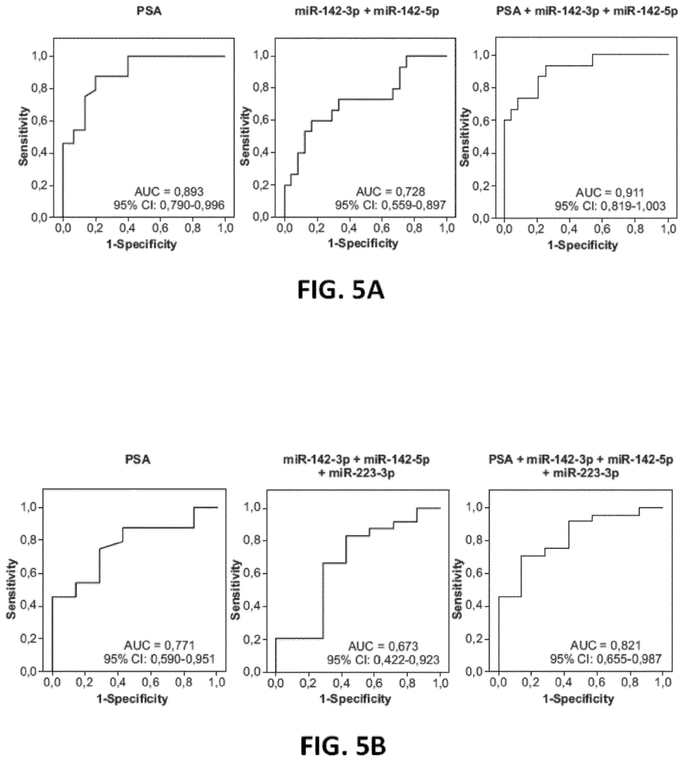

**Figure 5****:** MiRNA-based models as diagnostic classifiers. Receiver operating characteristic (ROC) analysis showing the predictive efficiency for distinguishing A) PCa from (HCt + BPH) and B) PCa from BPH samples, by using serum PSA, the model obtained from the combination of miRNAs (miR-142-3p, miR-142-5p and/or miR-223-3p) or the model that additionally includes PSA with the miRNAs (PSA, miR-142-3p, miR-142-5p and/or miR-223-3p) at the validation stage.

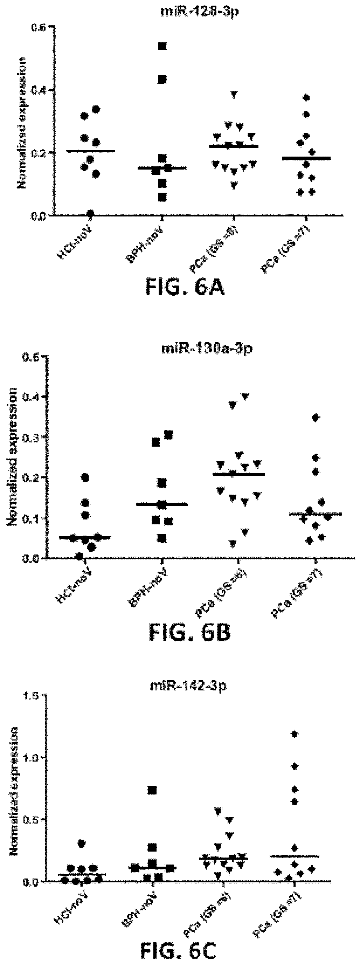

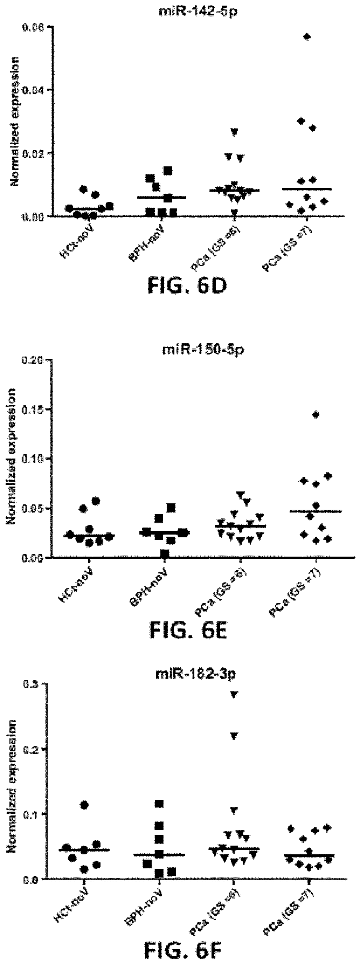

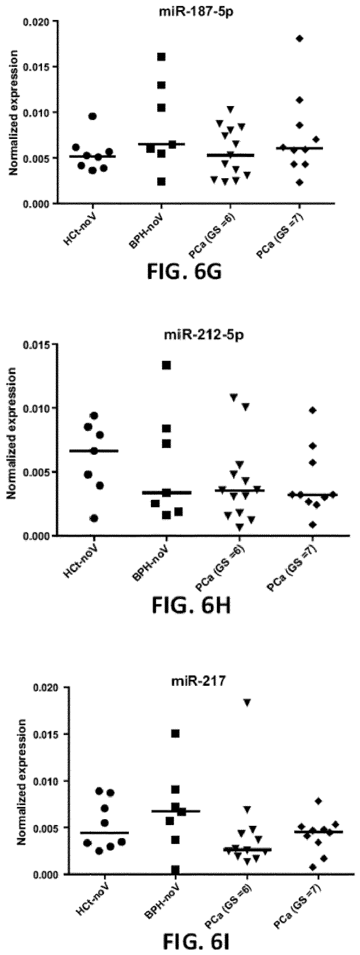

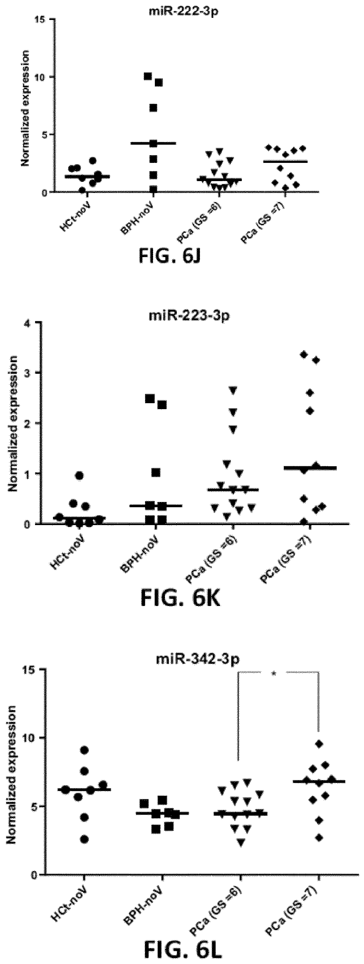

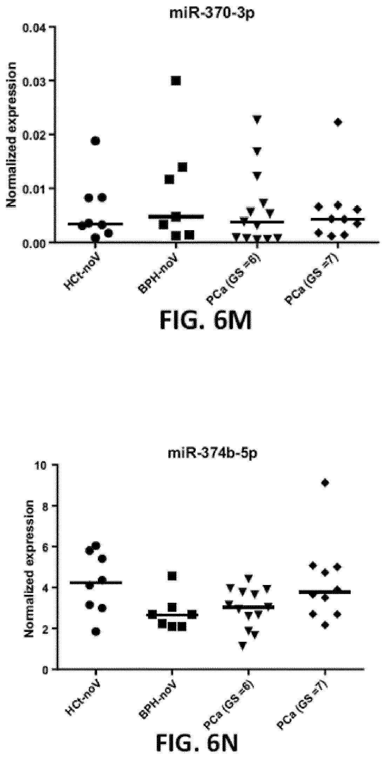

**Figure 6****:** Seminal plasma exosome miRNA levels in PCa samples with different severities of tumor defined by Gleason Score. Expression profiling, at the validating stage, of the 14 candidates miRNAs (A to N) in exosomes from semen of healthy controls (HCt), benign prostate hyperplasia-non vasectomised (BPH-noV), Gleason 6 classified prostate cancer [PCa (GS=6)] and Gleason 7 classified prostate cancer [PCa (GS=7)]. The horizontal bar displays the median cellular expression level. Significant differences between groups are indicated: *p<0.05; **p<0.01 (Mann Whitney U test).

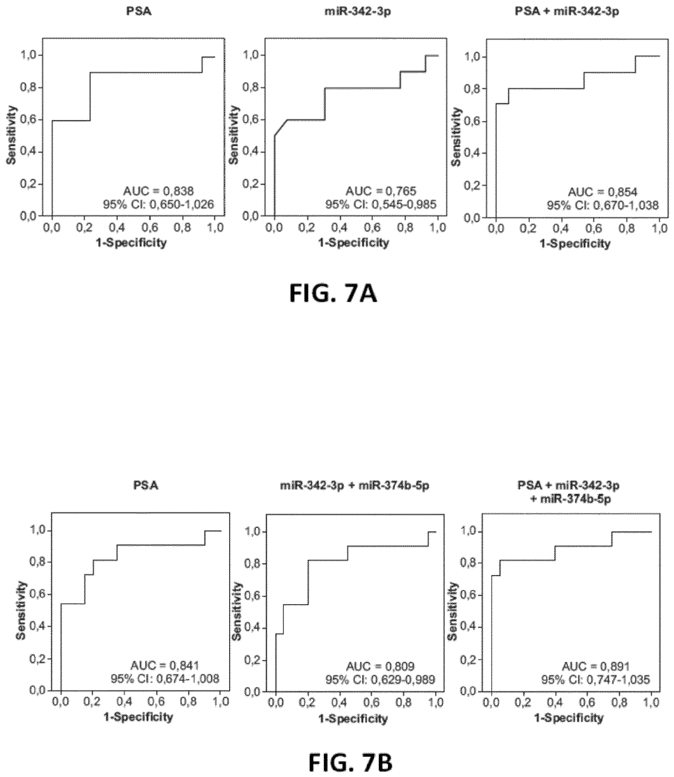

**Figure 7****:** MiRNA-based models as prognostic classifiers. Receiver operating characteristic (ROC) analysis showing the predictive efficiency for distinguishing A) Gleason 6 (GS6) from Gleason 7 (GS7) PCa samples and B) (BPH+GS6) samples from GS≥7 PCa samples, by using serum PSA and compared with the combined model [either PSA+miR-342-3p for A) and PSA+miR-342-3p+miR-374b-5p for B)] obtained at the validation stage.

### OBJECT OF THE INVENTION

The main object of the present invention is represented by the miRNAs as seminal fluid biomarkers for prostate cancer identified. In particular it is the object of the invention the miRNA biomarkers for the diagnosis and/or prognosis of prostate cancer in seminal fluid samples selected from miR-142-3p, miR-142-5p, miR-223-3p, miR-342-3p, miR-374b-5p or combinations thereof.

It is also an object of the invention an *in vitro* method for the diagnosis of prostate cancer in a subject or for discriminating prostate cancer from BPH in said subject comprising:
a) determining in a seminal fluid sample from said subject, the expression level of a miRNA selected from miR-142-3p, miR-142-5p, miR-223-3p, miR-342-3p, miR-374b-5p or combinations thereof,
b) comparing said expression levels with those of a healthy individual control group and/or with those of a BPH patient control group
wherein a differential expression of said miRNAs with respect to the healthy individual control group and/or with respect to the BPH patient control group results in the presence of prostate cancer in the subject.

Further, it is an object of the invention an *in vitro* method for discriminating the grade of a tumor in a subject suffering from prostate cancer comprising:
a) determining in a seminal fluid sample from said subject, the expression level of a miRNA selected from miR-142-3p, miR-142-5p, miR-223-3p, miR-342-3p, miR-374b-5p or combinations thereof,
b) comparing said expression levels with those of a BPH patient control group and/or with those of different grade patient control groups,
wherein a differential expression of said miRNAs with respect to the BPH patient control group and/or the assimilation of the expression with respect to one of the different grade patient control groups allows the discrimination of the grade of the prostate cancer in that subject.

Finally, it is an object of the invention a kit for the diagnosis and/or prognosis of prostate cancer in seminal fluid comprising a set of probes that selectively hybridizes with at least one of the miR-142-3p, miR-142-5p, miR-223-3p, miR-342-3p or miR-374b-5p.

### DETAILED DESCRIPTION OF THE INVENTION

The following definitions are provided to assist in the understanding and interpreting of the present invention:
**"MiRNA":** MicroRNAs (miRNAs) are conserved small non-coding RNAs (19-22 nucleotides) that act as negative post-transcriptional modulators of gene expression, affecting mRNA stability and translation by interacting with complementary sites on the 3'UTR of the target mRNAs. In the context of the present invention miR-142-3p, miR-142-5p, miR-223-3p, miR-342-3p, miR-374b-5p or combinations thereof are referred as the miRNAs of the invention.

**"Diagnosis":** in the context of the present invention it refers to the process for discriminating between patients with prostate cancer and healthy individuals or for discriminating patients with prostate cancer and BPH patients.

**"Prognosis":** in the context of the present invention it refers to the process for discriminating the severity of the cancer (grade of the tumor) in a subject suffering prostate cancer or a prostate disease such as BPH. The patients can be determined to have a non-severe disease (patients with BPH and GS6 grade tumor or less) or a severe disease (patients with GS≥7 grade tumor).

**"Prostate cancer (PCa)":** It refers to a malignant cell proliferative disease primarily affecting the prostate gland.

**"Benign Prostate Hyperplasia (BPH)":** it is a non-cancerous disease affecting the prostate by increasing its size. BPH may produce some symptoms similar to prostate cancer such as frequent urination, trouble in starting to urinate, weak stream, inability to urinate or loss of bladder control. Similarly to PCa, serum PSA test in BPH patients present elevated PSA levels. For this reason, in the context of this invention diagnosis and prognosis is also directed to discriminate prostate cancer patients and BPH patients with PSA levels between 4-10 ng/ml, these are patients which are in the grey zone.

**"Seminal fluid":** it generally refers to the fluids from different glands of the reproductive system such as prostate, seminal vesicles, Cowper glands, Litter glands, the epididymis and others which give rise all in conjunction with spermatozoids to formation of semen. In the context of the present invention, seminal fluid is particularly referred to prostate secretions or fluids containing prostate secretions like semen or seminal plasma.

**"Semen":** is a fluid formed by the conjunction of a cellular phase mainly containing spermatozoids and a liquid phase (seminal plasma) formed by the seminal fluids from different glands of the reproductive system such as prostate, seminal vesicles, Cowper glands, Litter glands, the epididymis and others.

**"Seminal plasma":** it refers to the liquid phase forming the semen. It is formed by secretions from all the glands of the male reproductive apparatus including the prostate.

**"Exosomes":** are cell-derived vesicles having a diameter between 30 and 100 nm. Exosomes contain a lot of miRNA molecules and they are abundant in seminal plasma. In the context of the present invention exosomes from seminal plasma represent a especially interesting source of miRNA for diagnostic/prognostic purpose.

**"Healthy individual control group":** It is the group of individuals representing the healthy population, that is, individuals neither having prostate cancer nor BPH. It can also be referred as negative healthy control.

**"BPH patient control group":** it is the group of individuals statistically representing patients with BPH It can also be referred as negative patient control.

**"Different grade patient control groups":** this expression embraces all the different control groups with prostate cancer in different grades that may be subjected to comparison with the individual or patient under study suffering from prostate cancer, in the prognostic method of the invention. In the context of this invention, the expression embraces "GS6 grade patient control group" and "GS≥7 grade patient control group".

**"GS6 grade patient control group":** It is the group of individuals statistically representing patients with GS6 grade prostate cancer.

**"GS≥7 grade patient control group":** It is the group of individuals statistically representing patients with a GS7 grade prostate cancer or higher.

**"Differential expression":** in the context of the comparative analysis between a patient under study and the different control groups in the diagnostic and/or prognostic methods of the invention, it refers to statistically significant differences in expression of the miRNAs of the invention in seminal fluid either by overexpression or underexpression. In particularly preferred embodiment, it refers to statistically significant differences in expression of the miRNAs of the invention present in exosomes from semen or seminal plasma samples.

**"Assimilation of the expression":** in the context of the comparative analysis between a patient under study and a certain control group in the diagnostic and/or prognostic methods of the invention, it refers to statistically non-significant differences in expression from the control group. This allows the identification of the clinical situation of the patient under study to that of the control group to which it was compared.

**"PSA screening test or PSA test":** it refers to the assays for determining the blood/serum levels of Prostate Specific Antigen (PSA), the prostate cancer biomarker commonly used for first screening in PCa diagnosis. The blood/serum PSA levels under 4ng/ml are considered as normal whereas levels over said value may be indicative of a prostate disease including prostatitis, BPH or prostate cancer.

**"Accuracy of the diagnosis":** it generally refers to sensibility and specificity of the method in a correct diagnosis.

**"Accuracy of discrimination":** it generally refers to sensibility and specificity of the method in a correct discrimination in a comparative analysis.

**"Isolation of exosomes":** in the context of the invention, it refers to the technique or group of techniques that comprises the treatment of a seminal fluid such as semen or seminal plasma to obtain a purified fraction enriched in exosomal vesicles. Any technique or group of techniques leading to a purified and enriched fraction in exosomal vesicles from seminal fluid such as semen or seminal plasma are contemplated within the scope of the invention. In a preferred embodiment of the invention, isolation of exosomes comprises differential centrifugation, followed by microfiltration and then ultracentrifugation.

**"Isolation of miRNA from the exosomes":** it generally refer to any technique or group of techniques that comprises the treatment of an exosomal fraction isolated from seminal fluid such as semen or seminal plasma to obtain the purified fraction enriched in the miRNAs contained within the exosomes. Any technique or group of techniques leading to a purified and enriched fraction in miRNAs from exosomes isolated from seminal fluid such as semen or seminal plasma are contemplated within the scope of the invention. Commercial kits like miRCURY RNA isolation kit-cell and plant (Exiqon; Denmark) may be used for isolation of miRNAs from exosomes.

**"Quantification of the expression levels of the miRNAs":** it generally refer to any technique or group of techniques that allows the quantification of a particular miRNA or group of miRNAs within an isolated miRNA fraction. Any technique or group of techniques leading to quantification of a purified and enriched fraction in miRNA from exosomes isolated from seminal fluid such as semen or seminal plasma are contemplated within the scope of the invention. In a particularly preferred embodiment of the invention, quantification of the expression levels of miRNAs is carried out by quantitative real-time RT-qPCR.

**"Differential centrifugation":** in the context of the present invention it refers to one or several centrifugation cycles that allows separating the cells, cell debris and apoptotic bodies from microvesicles and exosomes in seminal fluid samples such as semen or seminal plasma. In a particular and preferred embodiment of the invention it comprises a centrifugation step at 1000xg to 2000xg for 8 to 12 minutes, preferably 1500xg to 1700xg for 9 to 11 minutes, followed by a further centrifugation step at 10000xg to 20000xg for 8 to 12 minutes, preferably 15000xg to 17000xg for 9 to 11 minutes. This differential centrifugation steps are preferably carried out at low temperature that is between 1°C to 8°C, preferably 3°C to 5°C.

**"Microfiltration":** in the context of the present invention it refers to a filtration process of the supernatant obtained by subjecting to differential centrifugation of a seminal fluid samples such as semen and seminal plasma. Microfiltration allows the removal of apoptotic bodies still present in the supernatant and microvesicles >200nm. Microfiltration is preferably carried out through a microfilter of 0.3 µm to 0.15 µm diameter filters, preferably 0.3 µm to 0.2 µm.

**"Ultracentrifugation":** In the context of the present invention it refers to one or several ultrahigh speed centrifugation cycles that allows separating small extracellular vesicles (sEVs) fraction which mainly contain exosomes, from other fraction such as the microvesicles. Ina particular an preferred embodiment of the invention it comprises an ultracentrifugation step is on the filtrate at 90000xg to 110000xg for 1 to 4 hours, preferably 95000xg to 105000xg for 2 to 3 hours, more preferably at 100000xg for 2 hours. Ultracentrifugation is preferably carried out at low temperature, namely 1°C to 8°C, preferably 3°C to 5°C.

**"Grade of a tumor":** It is a way of classifying the prostate tumor of a patient in terms of the severity and aggressiveness of the cancer. The parameter used for characterizing the grade of the prostate cancer tumors is the so-called Gleason Score (GS). Prostate cancers with GS6 or less are generally considered non-severe cancers and prostate cancers with GS≥7 are generally deemed severe cancers.

**"Set of probes":** It refers to elements or group of elements, preferably of nucleic acid nature, that are capable of specifically binding or hybridizing with the miRNAs of the invention allowing their detection and eventually their quantification through any known technique such as in situ hybridization and preferably, by real time PCR. In a preferred embodiment, the set of probes may be primers, preferably LNA primers, or primers and probes for hybridization. The set of probes is part of the kit for PCa diagnosis and/or prognosis according to the invention. In a particular embodiment of the invention the set of probes detect at least one, but preferably all five of the following miRNA sequences:

| **miRNA** | **5'-3' sequence** |
|---|---|
| hsa-miR-142-3p | uguaguguuuccuacuuuaugga (SEQ ID NO 1) |
| hsa-miR-142-5p | cauaaaguagaaagcacuacu (SEQ ID NO 2) |
| hsa-miR-223-3p | ugucaguuugucaaauacccca (SEQ ID NO 3) |
| hsa-miR-342-3p | ucucacacagaaaucgcacccgu (SEQ ID NO 4) |
| hsa-miR-374b-5p | auauaauacaaccugcuaagug (SEQ ID NO 5) |

### MiRNAs as seminal biomarkers of prostate cancer

In a first aspect, the invention refers to miRNA biomarkers for the diagnosis and/or prognosis of prostate cancer in seminal fluid samples selected from miR-142-3p, miR-142-5p, miR-223-3p, miR-342-3p, miR-374b-5p or combinations thereof. These particular miRNAs or their combinations can be referred in the context of this invention as the miRNAs of the invention.

The present invention is based in the discovery that the miR-142-3p, miR-142-5p, miR-223-3p, miR-342-3p, miR-374b-5p present in seminal fluid, show a diagnostic and prognostic value either alone, by themselves and, preferably, in different combinations thereof.

The miRNAs of the invention have the following sequences:

| **miRNA** | **5'-3' sequence** |
|---|---|
| hsa-miR-142-3p | uguaguguuuccuacuuuaugga (SEQ ID NO 1) |
| hsa-miR-142-5p | cauaaaguagaaagcacuacu (SEQ ID NO 2) |
| hsa-miR-223-3p | ugucaguuugucaaauacccca (SEQ ID NO 3) |
| hsa-miR-342-3p | ucucacacagaaaucgcacccgu (SEQ ID NO 4) |
| hsa-miR-374b-5p | auauaauacaaccugcuaagug (SEQ ID NO 5) |

The diagnostic and prognostic value of the miRNAs of the invention as seminal fluid biomarkers of prostate cancer is even increased when used in combination with the results of the PSA screening test in blood/serum samples resulting in an increased sensitivity and sensibility.

In a particular embodiment of the invention, seminal fluid samples comprise semen or seminal plasma samples and, more preferably, they comprise exosomes isolated from semen or seminal plasma. The preferred use of exosomes isolated form semen and more particularly from seminal plasma as a source of miRNA biomarkers, is due to the fact that miRNAs are protected in exosomes by the exosomal membrane thus preventing them to degrade. This fact makes exosomal miRNAs as an ideal and more reliable source of miRNA biomarkers. This does not preclude that the miRNA of the invention can be identified either in total semen or seminal plasma.

Although, as explained the miRNAs of the invention have all by themselves diagnostic and/or prognostic value in preferred embodiments, a higher accuracy in the discrimination is achieved by the simultaneous analysis of the expression values of different combinations of the miRNAs of the invention.

Hence, in a preferred embodiment of the invention, the combination of miR-142-3p and miR-142-5p is used as seminal biomarker in the diagnosis of prostate cancer. This combination of miRNAs is especially useful in discriminating patients with prostate cancer from healthy individuals and patients with BPH. The sensitivity and sensibility of this combination of seminal biomarkers rises when used together with the PSA screening test results in blood/serum samples reaching up to 91.7 and 73.3% respectively.

In another preferred embodiment, the combination of miR-142-3p, miR-142-5p and miR-223-3p is used as seminal biomarker in the diagnosis of prostate cancer. This particular combination of miRNA is especially useful in discriminating patients with prostate cancer from patients with BPH. The sensitivity and sensibility of this combination of seminal biomarkers is also increased when used in together with the PSA screening test results in blood/serum samples reaching up to 91.7 and 42.9% respectively.

Another particularly preferred embodiment of the invention is represented by the miR-342-3p as seminal biomarker for the prognosis of prostate cancer. In particular, miR-342-3p allows for the discrimination between GS6 grade tumors and GS≥7 grade tumors in patients suffering from prostate cancer, that is, between non-severe and severe tumors. The combination of the miR-342-3p seminal biomarker with the results of the PSA screening test in blood/serum results in an even higher predictive accuracy in the prognosis of cancer disease.

Still, another preferred embodiment is represented by the combination of miR-342-3p and miR-374b-5p as seminal biomarker for the prognosis of prostate cancer. In particular, miR-342-3p allows for the discrimination between BPH patients or GS6 grade tumors, that is groups of men with non-severe prostatic disease, and GS≥7 grade tumors, *i.e.* patients with a more severe cancer disease. Again, the use of the combination of miR-342-3p and miR-374b-5p as seminal biomarker with the results of the PSA screening test in blood/serum results in an even higher predictive accuracy in the prognosis of cancer disease.

### Diagnostic method

A further aspect of the present invention is an *in vitro* method for the diagnosis of prostate cancer in a subject or for discriminating prostate cancer from BPH in said subject comprising:
a) determining in a seminal fluid sample from said subject, the expression level of a miRNA selected from miR-142-3p, miR-142-5p, miR-223-3p, miR-342-3p, miR-374b-5p or combinations thereof,
b) comparing said expression levels with those of a healthy individual control group and/or with those of a BPH patient control group
wherein a differential expression of said miRNAs with respect to the healthy individual control group and/or with respect to the BPH patient control group results in the presence of prostate cancer in the subject.

This general method can be referred along the description as the diagnostic method of the invention.

In a particular embodiment of the invention, once the results on the expression in seminal fluid of the biomarkers of the invention or their combinations have been obtained, the diagnostic method of the invention can actually combine said results with those obtained from the PSA screening test in a blood/serum sample to improve the accuracy of the diagnosis or discrimination.

The diagnostic method of the invention can be performed in seminal fluid samples and this is one of its main advantages as it is a non-invasive method for the diagnosis of prostate cancer. This implies that the determination of the expression levels of the miRNA can be carried out in semen or seminal plasma samples. In a preferred embodiment of the invention, the determination of the expression levels of the miRNA is carried out in the miRNAs contained in the exosomal vesicles of the seminal fluid samples. Exosomes represent a preferred source of miRNAs in the seminal fluid since miRNAs are kept well protected by the exosomal membranes which prevent their degradation. The use of the seminal fluid exosomes has therefore a positive impact in the reliability of the method.

In that sense, the method for *in vitro* diagnosis of prostate cancer of the invention in order to quantify the miRNAs of the invention preferably comprises:
a) Isolation of exosomes from the seminal fluid samples,
b) Isolation of miRNA from the exosomes, and
c) quantification of the expression levels of the miRNAs selected from miR-142-3p, miR-142-5p, miR-223-3p, miR-342-3p, miR-374b-5p or combinations thereof.

The isolation of exosomes form the seminal fluid can be carried out by any means of any technique which allows separating the exosomal fraction present in the seminal plasma of the seminal fluid samples. However, in a preferred embodiment, the isolation of exosomes from the seminal fluid samples comprises the steps of:
a) differential centrifugation,
b) microfiltration, and
c) ultracentrifugation.

For differential centrifugation (step a) a centrifugation step at 1000xg to 2000xg for 8 to 12 minutes, preferably 1500xg to 1700xg for 9 to 11 minutes, followed by a further centrifugation step at 10000xg to 20000xg for 8 to 12 minutes, preferably 15000xg to 17000xg for 9 to 11 minutes. This differential centrifugation steps are preferably carried out at low temperature that is between 1°C to 8°C, preferably 3°C to 5°C. This differential centrifugation allows pelleting cells, cell debris and apoptotic bodies whereas microvesicles and exosomes are kept in the supernatant.

Microfiltration is performed on the supernatant through filter of 0.3 µm to 0.15 µm diameter filters, preferably 0.3 µm to 0.2 µm. Microfiltration removed apoptotic bodies that may still be present and microvesicles >200nm.

Finally, an ultracentrifugation step is carried out on the filtrate at 90000xg to 110000xg for 1 to 4 hours, preferably 95000xg to 105000xg for 2 to 3 hours, more preferably at 100000xg for 2 hours. Ultracentrifugation is also preferably carried out at low temperature, namely 1°C to 8°C, preferably 3°C to 5°C. The ultracentifugation step results in sedimentation of the sEVs (small extracellular vesicles) fraction which mainly contains exosomes.

In other words, the isolation technique herein described allows the purification of the fraction enriched in exosomes (>90% of exosomes). This ensures and has the advantage that other fractions containing other extracellular vesicles, which presence can interfere in final results, are separated from the exosomal fraction.

For the quantification of the expression level of the miRNAs of the invention, isolation of the miRNA from the exosomes must be carried out first. This can be done by any RNA purification and isolation kit commercially available. Regarding miRNA quantification any method or technique that allows quantifying the expression levels of the isolated miRNA is applicable in the diagnostic method of the invention. In a particular and preferred embodiment the quantification of the expression levels of the miRNAs of the invention is carried out by quantitative real-time RT-qPCR.

A particularly preferred embodiment of the diagnostic method of the invention is represented by a method for the diagnosis of prostate cancer in a subject or for discriminating prostate cancer from BPH in said subject comprising:
a) determining in a seminal fluid sample from the subject, the expression level of a miRNA combination consisting of miR-142-3p and miR-142-5p,
b) comparing said expression levels with those of a healthy individual control group and/or with those of a BPH patient control group,
wherein a differential expression of said miRNAs with respect to the healthy individual control and/or with respect to the BPH patient control results in the presence of prostate cancer in the subject.

Another particularly preferred embodiment of the diagnostic method of the invention is represented by a method for the diagnosis of prostate cancer in a subject or for discriminating prostate cancer from BPH in said subject comprising:
a) determining in a seminal fluid sample from the subject, the expression level of a miRNA combination consisting of miR-142-3p, miR-142-5p and miR-223-3p,
b) comparing said expression levels with those of a healthy individual control group and/or with those of a BPH patient control group,
wherein a differential expression of said miRNAs with respect to the healthy individual control and/or with respect to the BPH patient control results in the presence of prostate cancer in the subject.

In these two embodiments the results of the combined miRNA analysis in seminal fluid, can be crossed or combined with those of the PSA screening test in a blood/serum sample to improve the accuracy of the diagnosis or discrimination.

### Prognostic method

A further aspect of the invention is represented by an *in vitro* method for discriminating the grade of a tumor in a subject suffering from prostate cancer comprising:
a) determining in a seminal fluid sample from said subject, the expression level of a miRNA selected from miR-142-3p, miR-142-5p, miR-223-3p, miR-342-3p, miR-374b-5p or combinations thereof,
b) comparing said expression levels with those of a BPH patient control group and/or with those of different grade patient control groups,
wherein a differential expression of said miRNAs with respect to the BPH patient control group and/or the assimilation of the expression with respect to one of the different grade patient control groups allows the discrimination of the grade of the prostate cancer in that subject.

This general method can be referred along the present description as the prognostic method of the invention.

In a particular embodiment of the invention, once the results on the expression in seminal fluid of the biomarkers of the invention or their combinations have been obtained, the prognostic method of the invention can actually combine said results with those of the PSA screening test in a blood sample to improve the accuracy of the prognosis or discrimination.

The prognostic method of the invention can be performed in seminal fluid samples and this is one of its main advantages as it is a non-invasive method for the prognosis of prostate cancer. The determination of the expression levels of the miRNA can hence be carried out in semen or seminal plasma samples. In a preferred embodiment of the invention, the determination of the expression levels of the miRNA is carried out in the miRNAs contained in the exosomal vesicles of the seminal fluid samples. Exosomes represent a preferred source of miRNAs in the seminal fluid since as explained miRNAs are kept well protected by the exosomal membranes preventing their degradation. The use of the seminal fluid exosomes has therefore a positive impact in the reliability of the prognostic method.

In that sense, for the prognostic method of the invention, the quantification of the miRNAs of the invention preferably comprises:
a) Isolation of exosomes from the seminal fluid samples,
b) Isolation of miRNA from the exosomes, and
c) quantification of the expression levels of the miRNAs selected from miR-142-3p, miR-142-5p, miR-223-3p, miR-342-3p, miR-374b-5p or combinations thereof.

The isolation of exosomes from the seminal fluid can be carried out by any means of any technique that allows purifying the exosomal fraction present in the seminal plasma of the seminal fluid samples. However, in a preferred embodiment, the isolation of exosomes from the seminal fluid samples comprises the steps of:
a) differential centrifugation,
b) microfiltration, and
c) ultracentrifugation.

For differential centrifugation a centrifugation step at 1000xg to 2000xg for 8 to 12 minutes, preferably 1500xg to 1700xg for 9 to 11 minutes, followed by a further centrifugation step at 10000xg to 20000xg for 8 to 12 minutes, preferably 15000xg to 17000xg for 9 to 11 minutes. This differential centrifugation steps are preferably carried out at low temperature that is between 1°C to 8°C, preferably 3°C to 5°C. Differential centrifugation allows pelleting cells, cell debris and apoptotic bodies whereas microvesicles and exosomes are kept in the supernatant.

Microfiltration is performed on the supernatant through filter of 0.3 µm to 0.15 µm diameter filters, preferably 0.3 µm to 0.2 µm. Microfiltration removes apoptotic bodies that may still be present and microvesicles >200nm.

Finally, an ultracentrifugation step is carried out on the filtrate at 90000xg to 110000xg for 1 to 4 hours, preferably 95000xg to 105000xg for 2 to 3 hours, more preferably at 100000xg for 2 hours. Ultracentrifugation is also preferably carried out at low temperature, namely 1°C to 8°C, preferably 3°C to 5°C. The ultracentifugation step results in sedimentation of the sEVs (small extracellular vesicles) fraction which mainly contains exosomes.

In other words, the isolation technique herein described allows the purification of the fraction enriched in exosomes (>90% of exosomes). This ensures and has the advantage that other fractions containing other microvesicles, which presence can interfere in final results, are separated from the exosomal fraction.

For quantification of the expression levels of the miRNAs of the invention, isolation of the miRNA form the exosomes must be carried out first. This can be done by any RNA purification and isolation kit commercially available. Regarding quantification any method or technique that is useful to quantify the expression levels of the isolated miRNA is valid and applicable in the prognostic method of the invention. In a particular and preferred embodiment, the quantification of the expression levels of the miRNAs in the prognostic method of the invention is carried out by quantitative real-time RT-qPCR.

Now a particular and preferred embodiment of the prognostic method of the invention is represented by a method for discriminating between GS6 grade tumor and GS≥7 grade tumor in a subject suffering from prostate cancer comprising:
a) determining in a seminal fluid sample from the subject, the expression level of the miR-342-3p miRNA,
b) comparing said expression levels with those of a GS6 grade patient control group,
wherein assimilation of the expression of miR-342-3p with respect to GS6 grade patient control group is indicative that the subject has a GS6 grade tumor whereas the non-assimilation of the expression of miR-342-3p with respect to GS6 grade patient control group is indicative that subject has a GS≥7 grade tumor.

For this particular embodiment of the prognostic method, the combination of the miRNA results in seminal fluid with those of the PSA screening test in a blood sample provides an improved accuracy in the discrimination.

Another particular and preferred embodiment of the invention is represented by a method for discriminating between GS≥7 grade tumor and BPH or GS6 grade cancer in a subject presenting PSA levels >4ng/ul comprising:
a) determining in a seminal fluid sample from the subject, the expression level of the combination of miR-342-3p and miR-374b-5p,
b) comparing the expression level of said combination with those of a GS6 grade patient control group or BPH patient control group,
wherein assimilation of the expression of said combination of miRNAs with respect to GS6 grade patient control group or BPH patient control group is indicative of the presence of a non-severe prostate cancer in that subject; whereas the non-assimilation of the expression of said combination of miRNAs with respect to GS6 grade patients control group or BPH patient control group is indicative of a severe prostate cancer in that subject.

### Kit

A final aspect of the invention is a kit for the diagnosis and/or prognosis of prostate cancer in seminal fluid comprising a set of probes that selectively hybridizes with at least one of the miR-142-3p, miR-142-5p, miR-223-3p, miR-342-3p or miR-374b-5p.

In addition, the set of probes the kit may contain means for detection and quantification of the target miRNAs.

The probes may be attached to a solid substrate such as an array or bead. Alternatively the probes are not attached. Once hybridization takes place detection and quantification may be carried out in an array-based system, a sequencing system, a PCR-based system or a bead-based system.

The set of probes are designed to detect at least one, but preferably all these five miRNA sequences

| **miRNA** | **5'-3' sequence** |
|---|---|
| hsa-miR-142-3p | uguaguguuuccuacuuuaugga (SEQ ID NO 1) |
| hsa-miR-142-5p | cauaaaguagaaagcacuacu (SEQ ID NO 2) |
| hsa-miR-223-3p | ugucaguuugucaaauacccca (SEQ ID NO 3) |
| hsa-miR-342-3p | ucucacacagaaaucgcacccgu (SEQ ID NO 4) |
| hsa-miR-374b-5p | auauaauacaaccugcuaagug (SEQ ID NO 5) |

### EXAMPLES

The identification and validation of the different miRNA biomarkers of the present invention was achieved on the basis of the results of a study which is the object of the next examples. For carrying out the study the following conditions and methods were used.

### METHODS

### Subjects of study

Patients and controls participating in the study were selected from men referred to the Urology Service of the Bellvitge Hospital and the Andrology Service of Fundacio Puigvert. The study was approved by the Institutional Review Board of both centres and all the participants signed a written informed consent.

Semen samples were obtained from 11 healthy individuals consulting for vasectomy (control group 1: HCt) and 31 individuals consulting for diagnosis of PCa who underwent routine prostate screening including PSA testing and DRE. Patients that presented moderate PSA levels (4-18 ng/ml) were selected with consent to undergo prostate biopsy. The latter group comprised: 24 men with biopsy-proven PCa including men who were previously successfully vasectomised (PCa-V, n=8) and non-vasectomised individuals (PCa-noV, n=16); and additionally, 7 non-vasectomised individuals with benign prostatic hyperplasia (BPH) or prostate enlargement (control group 2) who presented elevated PSA levels (>4 ng/ml) but no detectable cancer on biopsy.

For the subsequent analysis of exosome miRNA levels in semen, a two-stage (screening and validating stage) case control study (Fig. 1), was designed.

### Semen samples and exosome isolation

Semen samples were obtained by masturbation after 3-5 days of sexual abstinence. They were allowed to liquefy for 30 min at 37°C.
Isolation of exosomal vesicles was performed by differential centrifugation steps, microfiltration and ultracentrifugation. Briefly samples were centrifuged twice (1600 xg for 10 min, then 16000 xg for 10 min) at 4°C to obtain seminal plasma (supernatant). Seminal plasma was carefully collected and immediately stored at 80°C until use. Seminal plasma (200 µl) was first passed through a 0,22 µm filter to remove macromolecules and cell debris that they may have still contained. Subsequently, 9 ml of PBS was additionally filtrated. The resulting filtrate was ultracentrifuged at 100000 xg in a SW40 rotor for 2h at 4°C to sediment the sEVs, which mainly contain exosomes. The pellet was resuspended in 100 µl PBS and frozen at -80°C.

Nanoparticle tracking analysis was performed by NanoSight NS300 (Malvern Instruments Ltd; UK) showing an enrichment of particles with a size of <200nm, accounting for >90% of the particles recovered. Significant variations in the vesicle distribution by size and the morphological parameters was found between HCt and PCa groups, considering vesicle size mean (*p*=0,048), mode (*p*=0,024) and D50 (*p*=0,024) values.

### Small RNA-containing total RNA isolation

With the purpose of degrading the residual RNA outside the vesicles, the exosome suspension was treated with RNAse A (Qiagen NV; Germany) (100µg/ml final reaction concentration; 15 min at 37°C). Total RNA was obtained from exosomes using the miRCURY RNA Isolation Kit-Cell and Plant (Exiqon; Denmark). RNA concentration was calculated by using the QUBIT fluorometer and the Quant-iT RNA Assay kit (Invitrogen; California, USA). All RNA samples presented an OD 260/280 nm ratio ≥1.7 when using a Nanodrop UV-Vis spectrophotometer (Thermo Fisher Scientific; Massachusetts, USA).

### Exosomal miRNA quantitative real-time PCR profiling

For the miRNA screening, RNA was reverse transcribed (RT) using the miRCURY LNA™ Universal RT microRNA PCR, Polyadenylation and cDNA synthesis kit (Exiqon; Denmark). cDNA was diluted 50x and assayed in 10 µl PCR reactions according to the protocol for miRCURY LNA™ Universal RT microRNA PCR. Each miRNA was assayed once by quantitative real-time PCR (qPCR) using ExiLENT SYBR Green mastermix on the microRNA Ready-to-Use PCR Human panels I and II that include 634 mature miRNAs of miRBase (*www.**mirbase**.org*/) in a LightCycler® 480 Instrument (Roche; Switzerland). Experiments were conducted at Qiagen Genomic Services (Germany). Details and conditions of analysis of the amplification curves and efficiencies are described elsewhere [Barcelo M et al Hum Reprod 2018; Muñoz X. et al. Sci Rep 2015, 5:17991].
Next, to correct for potential overall differences in amount and quality between the samples, the raw data (Crossing Points Cp values) were normalised for each sample to the mean of the 50 most stable assays (mean 50) that were detected in all samples: dCp = mean 50 Cp - assay Cp. Those assays were previously selected as being the ones with the lowest coefficient of variation (CV < 0.017) of Cp values among samples in the study as well as showing no statistical differences in absolute expression levels between groups, either individually or for the mean value.

The relative quantitative method of 2^{dCp} was used to calculate the relative quantification (RQ) miRNA expression values.

### Validation of miRNA candidates by RT-qPCR analysis

First-stranded cDNA specific for miRNA was obtained by RT of 50 ng of exosomal RNA in 10 µl, using the Universal cDNA synthesis kit II (Exiqon; Denmark). For q PCR analysis, cDNA was diluted (12x) and assayed in 10 µl PCR reactions containing ExiLENT SYBR Green master mix (Exiqon; Denmark). Duplicate amplification reactions of individual assays (LNA™-enhanced miRNA qPCR primers) were carried out on a Lightcycler® 96 Instrument (Roche; Switzerland). Target miRNA expression for exosomes in semen samples was calculated relative to the mean expression value of miR-30e-3p and miR-126-3p, chosen from the exosomal miRNA qPCR profiling study as being among the most stable miRNAs. The RQ values were calculated using the 2^{dCp} strategy. The same procedure was applied to determine tissue expression profiling of miRNA candidates with some modifications: 20 ng of tissue RNA were reverse transcribed and cDNA was diluted 80x.

### Determining in silico target genes of miRNAs

Identification of target genes and pathways potentially altered by the miRNA signature was performed using the miRNet web-based platform [Fan Y et al. Nucleic Acids Res 2016, 44:W135-141]. The miRNet predicted targets are determined experimentally as part of larger experiments such as microarray, RNA-seq, qPCR and PAR-CLIP, and immunoprecipitation method for identifying the binding sites of cellular RNA-binding proteins (RBPs) and miRNA-containg ribonucleoportein complexes (miRNPs) and each identified target gene is supported by the experiment name and PubMed literature. Additional analysis of the miRNA expression in the TCGA dataset and functional analysis was performed by using this platform.

### Statistical analysis

The non-parametric Kruskal-Wallis test was used to analyze the differences in clinical data and absolute expression levels of reference genes. Unpaired two-tailed Student's t test was used to analyze the differences in relative expression of miRNAs between groups in the miRNA profiling study and the Benjamini-Hochberg procedure for multiple testing corrections, using a maximum discovery rate of 5%, was applied to calculate the False Discovery Rate (FDR) for each of the p-values obtained. The non-parametric Mann-Whitney U-test was used to evaluate differences in relative expression of selected miRNAs between groups. Pearson product-moment correlation coefficients (r) were calculated to determine the correlation between the miRNA RQ values and the various parameters in semen analysis, blood analysis or prostate biopsy in patient groups and controls. Multivariate binary logistic regression (backward stepwise, conditional, method) and receiver operating characteristic (ROC) curve analysis of the RQ values was used to distinguish the samples showing malignancy of prostate tumor. Accuracy was measured as the area under the ROC curve (AUC). All data analyses were performed using the SPSS software version 15.0 (SPSS Inc.; IBM; IL, USA). A p-value ≤0.05 was considered significant.

### Example 1: screening of miRNA in seminal fluid show altered exosomal miRNA profile in Benign prostate hyperplasia and malignant prostate tumor

The clinicopathological characteristics of patients are presented in Figure 2.

Cases (PCa) and benign prostatic hyperplasia (BPH) controls mostly (25 out of 31 individuals: 80.6%) fall within the serum PSA diagnostic "grey zone" (4-10 ng/ml). These two groups were similar regarding age, whereas pre-biopsy PSA in PCa-noV was significantly increased (*p*=0.02) when compared to BPH controls. The mean age of the healthy controls (HCt) differed significantly from the other groups (*p*<0.001). Most cases (23 out of the 24 individuals) showed mild disease, with PCa in early stages (GS 6 or 7). The study was divided into two phases (Fig. 1). In the first stage of the study, a high throughput analysis of the level of expression of 634 human miRNAs was performed in order to identify the global exosomal miRNA changes in seminal fluid associated with malignant PCa. All the BPH and PCa individuals included in this first phase presented PSA levels within the 4-6 ng/ml range.

The expression behavior of 400 miRNAs (63%) between groups was statistically analyzed. The rest of the miRNAs were excluded from analysis as in some cases (n=165) no amplification values (Cp value>38) were obtained in either group of the study, suggesting that the transcript levels of these miRNAs were beneath the detection threshold of the technique, and in other cases (n=69) poor amplification efficiency across samples (missing expression values for eight out of the twelve samples) was obtained (data not shown).

The presence of 392 miRNAs was confirmed in HCt samples; 42 of these miRNAs were not expressed in PCa-V samples, although they presented detectable expression in PCa-noV, suggesting that they are preferentially expressed in testis and/or epididymis.

The study revealed significantly altered expression levels of 100 miRNAs in PCa and/or BPH compared with HCt individuals (uncorrected *p*≤0.05). Fifty of them were significantly under-expressed in PCa-V individuals, but not in PCa-noV, suggesting that the loss of expression is a consequence of the vasectomy procedure and does not originate from the presence of the tumor. As previously described in obstructive azoospermia [Barcelo M et al. Hum Reprod 2018], many of these miRNAs that present a reduced in expression in semen from vasectomised PCa individuals map to miRNA clusters in chromosome 19 and chromosome X.

Among the remaining differentially expressed miRNAs, 42 of them were altered in expression in BPH when compared to HCt controls (28 over-expressed and 14 under-expressed); 21 miRNAs were dysregulated in PCa-noV compared to HCt controls (15 over-expressed and 6 under-expressed); six miRNAs were shared among PCa-noV and PCa-V (either up-regulated miR-142-5p, miR-212-5p, miR-182-3p, or down-regulated miR-342-3p, miR-151a-3p, miR-217), and three of them were shared among BPH and PCa samples (miR-142-5p, miR-151a-3p, miR-342-3p). Additionally, when PCa were compared to BPH semen samples we found 11 up-regulated and 16 down-regulated miRNAs. Nevertheless, no miRNA passed the FDR correction.

### Example 2: validation of miRNAs and candidate selection

Given the profiling results in the miRNA panels, we proceeded to validate several miRNAs as candidate biomarkers of PCa malignancy in a larger cohort of samples (second phase of the study; Fig. 1). Several miRNAs were candidates for validation based on the following criteria: we selected those miRNAs that presented ≥ 1.5 fold difference in expression between groups, a Cp value ≤36 in any of the groups, and additionally, the expression in PCa-V should represent >20% of expression in PCa-noV. Out of 39 miRNAs that fulfilled the criteria, 14 were finally selected for validation (up-regulated: miR-142-5p, miR-128-3p, miR-142-3p, miR-223-3p, miR-212-5p, miR-182-3p, miR-130a-3p, miR-222-3p, miR-187-5p, miR-370-3p; down-regulated: miR-342-3p, miR-374b-5p, miR-217, miR-150-5p). Interestingly, these included five out of the six miRNAs deregulated in PCa-noV and PCa-V samples and three miRNAs (miR-212-5p, miR-217, miR-222-3p) that presented significant differences in expression between PCa and BPH phenotypes whereas one miRNA (miR-187-5p) was differentially expressed between PCa-noV and BPH.

In order to determine the expression level of each miRNA in the different organs of the reproductive tract, the expression of the 14 miRNAs was first tested in testis, epididymis, prostate and SP (two samples each). Controls of pathological prostate (BPH and PCa tissue) and lymphocytes, the latter as external control cells, were also included (two samples each). We were able to corroborate that all miRNAs tested were expressed in the prostate and most of them were altered in expression in the BPH and PCa prostate samples (Fig. 3).

Therefore, in the validation stage of the study (Fig. 1), these particular 14 miRNAs were individually reanalysed in a subsequent set of semen samples by RT-qPCR (Fig. 4). The expression tendencies between groups of the 14 miRNAs analysed were mostly conserved between the miRNA panels and the RT-qPCR individual assays.

### Example 3: Diagnostic and prognostic performance of single miRNAs and development of combined miRNA-based diagnostic/prognostic classifiers for PCa.

First, the validation results showed that the expression values of miR-130a-3p (*p*=0.015), miR-142-3p (*p*=0.006), miR-142-5p (*p*=0.006) and miR-223-3p (*p*=0.003) were statistically different between PCa and HCt groups (Fig. 4). Specifically, when PCa samples were divided into PCa-V and PCa-noV groups, miR-130a-3p (*p*=0.005), miR-142-3p (*p*=0.019), miR-142-5p (*p*=0.019), miR-223-3p (*p*=0.009) and miR-222-3p (*p*=0.045) expression values were statistically different between PCa-noV and HCt groups, whereas miR-142-3p (*p*=0.010), miR-142-5p (*p*=0.010), miR-223-3p (*p*=0.010) and miR-222-3p (*p*=0.015) expression values were statistically different between PCa-V and HCt groups. Interestingly, three of these miRNAs were statistically different in expression in the presence of malignant tumor in the prostate (PCa group) when compared with the absence of a tumor (HCt + BPH group): miR-142-3p (*p*=0.012), miR-142-5p (*p*=0.015) and miR-223-3p (*p*=0.020). Strikingly, we found that miR-142-3p, miR-142-5p and miR-223-3p were indeed over-expressed in PCa and BPH tissue when compared with healthy tissue (Fig. 3) suggesting that the over-expression of these miRNAs in prostate fits perfectly with a quantified higher concentration in semen exosomes and, thus may well be a reflection of the prostate health.

The expression values of these three miRNAs in semen exosomes resulted in good predictive accuracy [miR-142-3p (AUC: 0.739, *p*=0.013), miR-142-5p (AUC: 0.733, *p*=0.015) and miR-223-3p (AUC: 0.722, *p*=0.021)] to discriminate PCa from (HCt+BPH) control individuals, suggesting that they have a potential use as biomarkers of the presence of malignant cells in the prostate. As a comparison, the ROC curve analysis of blood PSA levels was also determined (AUC: 0.893, *p*<0.001), resulting in a sensitivity of 91.7% and specificity of 60% when used as a classifier for PCa in the study. To determine if a multiplex model could improve performance over single biomarkers for discriminating PCa from non-malignant samples, the three previously significant miRNAs were analysed in a multivariate logistic regression analysis. Interestingly, this analysis resulted in a model that included the miR-142-3p and miR-142-5p expression values (AUC: 0.728, *p*=0.018). In this case, the sensitivity and specificity for predicting the PCa samples were 83.3% and 60% respectively. Strikingly, when compared with PSA, a moderate increased value of specificity (Sn: 91.7 and Sp: 73.3%) was obtained when PSA+miR-142-3p+miR-142-5p were included in the model (AUC: 0.911, *p*<0.001) (Fig. 5A).

Additionally, the same analysis was performed in samples from individuals who presented PSA levels ≥4ng/ml in order to discriminate PCa from BPH individuals. In this case blood PSA levels resulted in a high sensitivity of 100% but in a low specificity of 14.3% for PCa as previously described (AUC: 0.771, *p*=0.032), the same as the ones obtained when the three miRNAs (miR-142-3p+miR-142-5p+miR-223-3p) were included in the model (AUC: 0.673, *p*=0.171). When all four variables (PSA+miR-142-3p+miR-142-5p+miR-223-3p) were introduced in the analysis it resulted in a model with higher accuracy (AUC: 0.821, *p*=0.011) and a better use for diagnosis: sensitivity of 91.7% and specificity of 42.9% (Fig. 5B).

Furthermore, the same type of analysis was performed in order to determine if a multiplex miRNA model could reflect the severity or degree of PCa affectation. We found miR-342-3p can distinguish (AUC: 0.765; *p*=0.032) between PCa samples with GS6 and those with GS7 in the biopsy (Fig. 6). Again, an increased value of true positive and negative rates for predicting a higher PCa Gleason score (60 and 100% respectively; AUC 0.854, *p*=0.004) was obtained when miR-342-3p+PSA was included in the model, much better than the ones obtained using single biomarkers: PSA (60 and 84.6%; AUC 0.838, *p*=0.006) or miR-342-3p (60 and 76.9%) (Fig. 7A).

What is more, miR-342-3p (AUC: 0.800; *p*=0.006; Sn: 63.6%; Sp: 90%), miR-374b-5p (AUC: 0.768; *p*=0.015; Sn: 45.5%; Sp: 90%) and the combination of both miRNAs (AUC: 0.809; *p*=0.005; Sn: 54.5%; Sp: 80%) permit the discrimination between a group of men with GS≥7 and a group of men presenting PSA levels >4ng/ml without cancer or with GS6. These results are similar to the results obtained for PSA (AUC: 0.841, *p*=0.002; Sn: 54.5%; Sp: 90%), indicating that these markers have prognostic value. The combined model (PSA+miR-342-3p+miR-374b-5p) resulted in a higher predictive accuracy (AUC: 0.891; *p*<0.001) with a clinically useful sensitivity and specificity (81.8% and 95% respectively) (Fig. 7B).

## Claims

1. MiRNAs as biomarkers for the diagnosis and/or prognosis of prostate cancer in seminal fluid samples selected from miR-142-3p, miR-142-5p, miR-223-3p, miR-342-3p, miR-374b-5p or combinations thereof.

2. MiRNAs according to claim 1 wherein the seminal fluid comprises semen or seminal plasma or where seminal fluid samples comprises exosomes isolated from semen or seminal plasma.

3. MiRNAs according to claim 1 or 2 for use in combination with PSA levels in a blood/serum sample for the diagnosis and/or prognosis of prostate cancer.

4. *In vitro* method for the diagnosis of prostate cancer in a subject or for discriminating prostate cancer from BPH in said subject comprising:
a) determining in a seminal fluid sample from said subject, the expression level of a miRNA selected from miR-142-3p, miR-142-5p, miR-223-3p, miR-342-3p, miR-374b-5p or combinations thereof,
b) comparing said expression levels with those of a healthy individual control group and/or with those of a BPH patient control group,
wherein a differential expression of said miRNAs with respect to the healthy individual control group and/or with respect to the BPH patient control group results in the presence of prostate cancer in the subject.

5. Method according to claim 4 comprising the combination of the miRNA results with those of the PSA screening test in a blood/serum sample to improve the accuracy of the diagnosis or discrimination.

6. Method according to any of claims 4 or 5 wherein the miRNAs are contained in exosomal vesicles of the seminal fluid sample.

7. Method according to claim 4 where the determination of the expression levels of the miRNAs comprises:
a) Isolation of exosomes from the seminal fluid samples,
b) Isolation of miRNA from the exosomes, and
c) quantification of the expression levels of the miRNAs selected from miR-142-3p, miR-142-5p, miR-223-3p, miR-342-3p, miR-374b-5p or combinations thereof.

8. Method according to claim 7 wherein the isolation of exosomes from the seminal fluid samples comprises the steps of:
a) differential centrifugation,
b) microfiltration, and
c) ultracentrifugation.

9. Method according to claim 7 or 8 wherein quantification of the expression levels of the miRNAs is carried out by quantitative real time RT-qPCR.

10. Method according to any one of claims 4 to 9 comprising:
a) determining in a seminal fluid sample from the subject, the expression level of a miRNA combination consisting of miR-142-3p and miR-142-5p,
b) comparing said expression levels with those of a healthy individual control group and/or with those of a BPH patient control group,
wherein a differential expression of said miRNAs with respect to the healthy individual control and/or with respect to the BPH patient control results in the presence of prostate cancer in the subject.

11. Method according to any one of claims 4 to 9 comprising:
a) determining in a seminal fluid sample from the subject, the expression level of a miRNA combination consisting of miR-142-3p, miR-142-5p and miR-223-3p,
b) comparing said expression levels with those of a healthy individual control group and/or with those of a BPH patient control group,
wherein a differential expression of said miRNAs with respect to the healthy individual control and/or with respect to the BPH patient control results in the presence of prostate cancer in the subject.

12. Method according to claim 10 or 11 comprising the combination of the miRNA results with those of the PSA screening test in a blood/serum sample to improve the accuracy of the diagnosis or discrimination.

13. *In vitro* method for discriminating the grade of a tumor in a subject suffering from prostate cancer comprising:
a) determining in a seminal fluid sample from said subject, the expression level of a miRNA selected from miR-142-3p, miR-142-5p, miR-223-3p, miR-342-3p, miR-374b-5p or combinations thereof,
b) comparing said expression levels with those of a BPH patient control group and/or with those of different grade patient control groups,
wherein a differential expression of said miRNAs with respect to the BPH patient control group and/or the assimilation of the expression with respect to one of the different grade patient control groups allows the discrimination of the grade of the prostate cancer in that subject.

14. Method according to claim 13 comprising the combination of the miRNA results with those of the PSA screening test in a blood/serum sample to improve the accuracy of the discrimination.

15. Method according to any of claims 13 or 14 wherein the miRNAs are contained in exosomes of the seminal fluid sample.

16. Method according to claim 13 where the determination of the expression levels of miRNAs comprises:
a) Isolation of exosomes from the seminal fluid samples,
b) Isolation of miRNA from the exosomes, and
c) quantification of the expression levels of miRNAs selected from miR-142-3p, miR-142-5p, miR-223-3p, miR-342-3p, miR-374b-5p or combinations thereof.

17. Method according to claim 16 wherein the isolation of exosomes from the seminal fluid samples comprises the steps of:
a) differential centrifugation,
b) microfiltration, and
c) ultracentrifugation.

18. Method according to claim 16 or 17 wherein quantification of the expression levels of the miRNAs is carried out by quantitative real time RT-qPCR.

19. Method according to any one of claims 13 to 18 for discriminating between GS6 grade tumors and GS≥7 grade tumors in a subject suffering from prostate cancer comprising:
a) determining in a seminal fluid sample from the subject, the expression levels of the miR-342-3p miRNA,
b) comparing said expression levels with those of a GS6 grade patient control group,
wherein assimilation of the expression of miR-342-3p with respect to GS6 grade patients control groups is indicative that the subject has a GS6 grade tumor whereas the non-assimilation of the expression of miR-342-3p with respect to GS6 grade patients control groups is indicative that subject has a GS≥7 grade tumor.

20. Method according to any one of claims 13 to 18 for discriminating between GS≥7 grade tumors and BPH or GS6 grade cancer in a subject presenting PSA levels >4ng/ul comprising:
a) determining in a seminal fluid sample from the subject, the expression level of the combination of miR-342-3p and miR-374b-5p,
b) comparing the expression level of said combination with those of a GS6 grade patient control group or BPH patient control group,
wherein assimilation of the expression of said combination of miRNAs with respect to GS6 grade patient control group or BPH patient control group is indicative of the presence of a non-severe prostate cancer in that subject; whereas the non-assimilation of the expression of said combination of miRNAs with respect to GS6 grade patients control group or BPH patient control group is indicative of a severe prostate cancer in that subject.

21. Method according to claim 19 or 20 comprising the combination of the miRNA-results with those of the PSA screening test in a blood/serum sample to improve the accuracy of the discrimination.

22. A kit for the diagnosis and/or prognosis of prostate cancer in seminal fluid comprising a set of probes that selectively hybridizes with at least one of the miR-142-3p, miR-142-5p, miR-223-3p, miR-342-3p or miR-374b-5p.
